# EUROPEAN PATENT APPLICATION

(11) **EP 3 970 672 A1**
(43) Date of publication of application: **23.03.2022**
(21) Application number: 21197858.0
(22) Date of filing: 20.09.2021
(51) Int. Cl.: A61F 5/01, B25J 9/00, B25J 18/06, A61H 1/02

(54) **SEMI-PASSIVE EXOSKELETON**

(30) Priority: 22.09.2020 PL 43538720
(71) Applicant: Icarion Sp. z.o.o. Sp.k., 31-539 Kraków (PL)
(72) Inventor: GACZOREK, Bartlomiej, 62-035 Kraków (PL); FRAS, Jan, 62-035 Kurnik (PL)
(74) Representative: Adamczyk, Piotr

(57) **Abstract**

The exoskeleton has the first holder (1) for the first body part (2), the second holder (3) for the second body part (4), the articulated connection (5) for both holders (1,3), at least one pneumatic artificial muscle (6) connecting both holders (1, 3) with its ends, and an adjustable source of overpressure supplying the artificial muscles (6). The artificial muscle (6) comprises at least one flexible working chamber (7) in the form of a round tube section made of a flexible plastic with a non-stretchable braid (8) closed at both ends. The braid (8) of the working chamber (7) is sunk into its side wall and is made of an inextensible thread running along a helical line with the axis coinciding with the axis of the working chamber (7). The diameter (D2) of the braid (8) is from 100% to 200% of the inner diameter (D1) of the working chamber (7). The pitch of the braid (8) is from 3% to 30% of its diameter (D2) and the thread thickness of the braid (8) is from 1% to 5% of its diameter (D2). The outer diameter (D3) of the working chamber (7) is from 100% to 200% of the diameter (D2) of its braid (8). The length of each working chamber (7) in a free state is from 100 to 6000% of the diameter (D2) of its braid (8), while the hardness of the plastic forming the working chamber (7) is from 10 to 30 according to the Shore A scale.

## Description

The subject of the invention is a semi-passive exoskeleton, supporting or replacing the action of natural human muscles with the help of artificial pneumatic muscles.

The exoskeleton is an orthopedic device that supports the mobility of the part of a body on which it is attached. Mainly known and used are passive exoskeletons which support the natural skeleton in transferring the load acting on the patient's body and active skeletons which are equipped with various types of actuators and can therefore support or even replace the work of the patient's muscles. From some time now, the so-called semi-passive exoskeletons are known, combining the functions of a passive and active exoskeleton, thanks to the use of a specific type of actuators and their control. As the number of users of exoskeletons increases, the need to quickly adjust the exoskeleton to a specific patient and the degree of advancement in using such a device becomes more and more important. An example of a hand skeleton is disclosed in EP1949873A1. This device includes a holder mounted on an arm and a holder mounted on a forearm of an assisted hand. Both of these holders are articulated in the area of the elbow joint. Movement support in such an exoskeleton is possible thanks to the use of known pneumatic actuators, called pneumatic muscles or McKibben's artificial muscles. McKibben's muscles have a flexible, for example rubber, chamber surrounded by a mesh, non-stretchable braid. McKibben's muscle behaves similar to the natural biceps muscles. Upon exposure to pressure, the artificial muscle swells and reduces its length, attracting the parts it is attached to. An example of McKibben's artificial muscle is disclosed in WO2017/047208.

Also known are the so-called soft manipulators, i.e. pneumatic actuators having working chambers in the form of a round tube made of a flexible material, e.g. silicone. In the side wall of such a chamber there is a braid made of an inextensible thread, which runs along a helical line with an axis coinciding with the axis of the working chamber. Due to the mentioned braid, the tube of the working chamber supplied with compressed air can only extend. An example of such a manipulator and a method of making it are disclosed in PL229119B1.

The aim of the invention was to develop a construction of a semi-passive exoskeleton with greater versatility than before.

This aim is fulfilled by the exoskeleton according to the invention, which comprises the first holder for the first body part, the second holder for the second body part, an articulated joint of the two holders, at least one pneumatic artificial muscle, and an adjustable source of excess pressure supplying the working chambers interior of the artificial muscle. The artificial muscle comprises at least one flexible working chamber with a non-stretchable braid and connecting both holders at its ends. The invention consists in the fact that the working chamber of the artificial muscle is in the form of a round tube section made of a flexible material, closed at both ends. The braid of the working chamber is sunk into its side wall and is made of an inextensible thread running in a helical line coaxial with the axis of the working chamber. The braid diameter ranges from 100% to 200% of the inner diameter of the working chamber tube. The braid pitch is from 3% to 30% of its diameter, and the thickness of the braid thread is from 1% to 5% of its diameter. The working chamber outer diameter is from 100% to 200% of the braid diameter. The length of each working chamber in a free state ranges from 100 to 6000% of its braid diameter. The hardness of the plastic forming the working chamber is from 10 to 30 according to the Shore A scale.

In one variant of the invention, the first exoskeleton holder is in the form of an openwork, side-open sleeve surrounding the arm, and the second exoskeleton holder is an openwork, side-open sleeve surrounding the forearm. Both mentioned holders are made of an elastic plastic and connected to each other by means of two ball joints located in the elbow area.

In next variant of the invention, the plastic material of the exoskeleton holders has a Young's modulus ranging from 1500 to 1900 MPa.

In next variant of the invention, the exoskeleton holders are made of polyamide. In next variant of the invention, the exoskeleton comprises two artificial muscles. Each of these artificial muscles has at least two parallel, substantially identical working chambers. The interiors of the working chambers of one muscle are connected to each other, and their first ends are connected to the first attachment means that secures this muscle to the first exoskeleton holder. The second ends of the working chamber of one muscle are connected to the second attachment means securing the muscle to the exoskeleton other holder. In next variant of the invention, the exoskeleton also has at least two artificial muscles, and each of these artificial muscles has at least two working chambers, the interiors of which are connected to each other. In such an exoskeleton, the first ends of the working chambers of one muscle are connected to the first attachment means securing the muscle to the first holder, while the second ends of the working chambers of one muscle have separate second attachment means securing the muscle to the other holder.

In next variant of the invention, each artificial muscle of the exoskeleton has two working chambers of silicone with hardness 10 according to Shore A scale. The inner diameter of each working chamber is 8 mm and its outer diameter is 12 mm. The working chamber length in a free state ranges from 40 to 120 mm and each working chamber braid diameter is 10 mm.

In another variant of the invention, the first and second artificial muscle attachment means are in the form of a seat with a blind T-slot in which a circular mushroom-shaped catch is received, located on the exoskeleton holder. This slot is closed on the artificial muscle action side and the axis of said T-slot coincides with this artificial muscle action axis.

In yet another variant of the invention, the articulated joint of the first and second exoskeleton holders has a stop and each holder has securing strips to prevent it from disengaging from the limb it surrounds.

The exoskeleton according to the invention is characterized by a lower weight and dimensions compared to known solutions, which translates into greater user comfort. The smaller size of the exoskeleton allows it to be worn under clothing and the lower weight increases the wearing time without feeling tired. The invention increases patient safety since the support force never exceeds the preload of the pneumatic actuator, regardless of the supply pressure level. In the event of a failure resulting in loss of the compressed air supply, the exoskeleton assisted arm is held in a bent state. The invention also provides energy savings, as the work is only performed in one direction and the return is due to the energy stored in the form of elastic energy. Moreover, the proper balance of the mechanism allows the reduction of energy expenditure in the active part of the cycle, because in case of a hand exoskeleton, its straightening is assisted by gravity. The construction of the exoskeleton according to the invention greatly simplifies the adjustment of the support degree, which is carried out by adjusting the stretch of the artificial muscle. This simplifies the use of the invention in rehabilitation, as the exoskeletons used in rehabilitation require constant adjustment, adjusting the support degree to the current condition of a patient. Since the main control medium of the device according to the invention is compressed air, in stationary solutions, it is possible to use a fixed, e.g. hospital, compressed air installation. The exoskeleton can be controlled by grading the pressure on the manipulator rather than by moving it, so that the patient's transmission of signals to the controller takes place with minimal body movement. The semi-elastic material of the elements connecting the exoskeleton with the patient's body and articulated joint of these elements provides a better fit than before to the anatomically natural movement and compensation of slight misalignments. The possibility of modifying the artificial muscles by selecting the number and size of their working chambers and the nature of the plug allows to reduce the dimensions of the exoskeleton and better anatomically fit it to the supported body part.

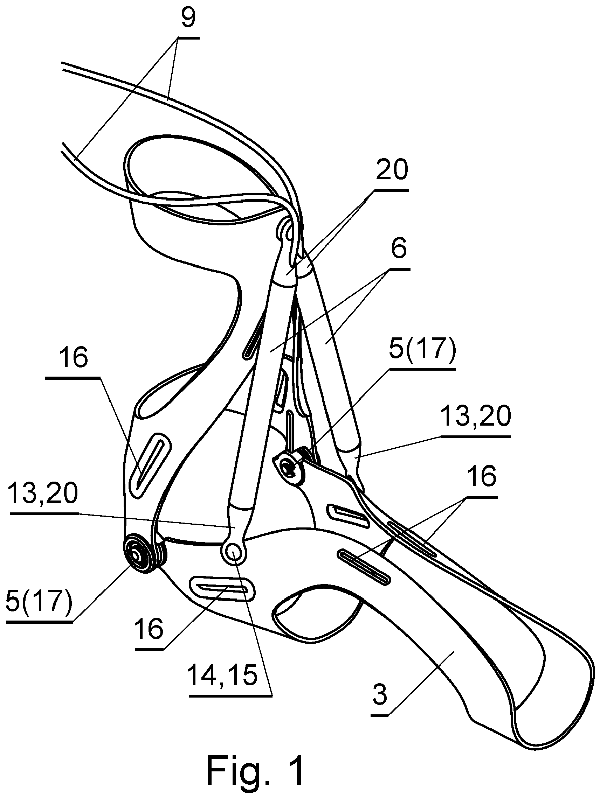

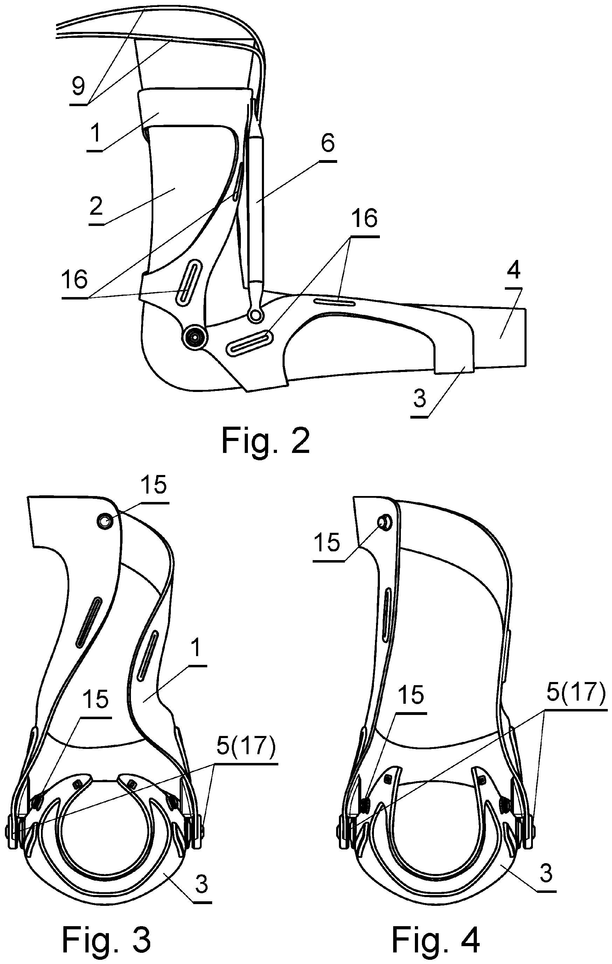

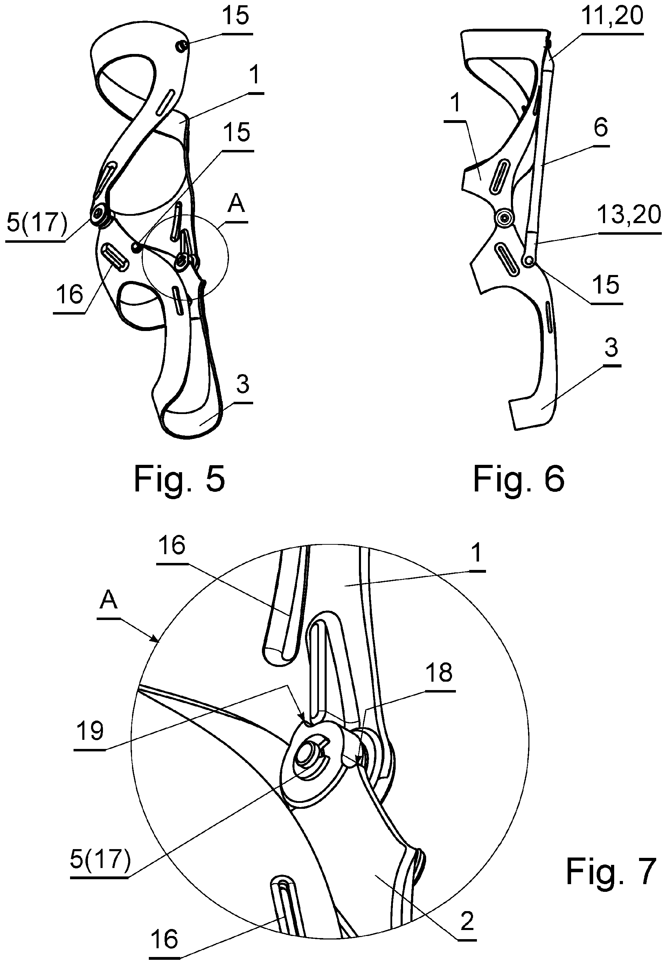

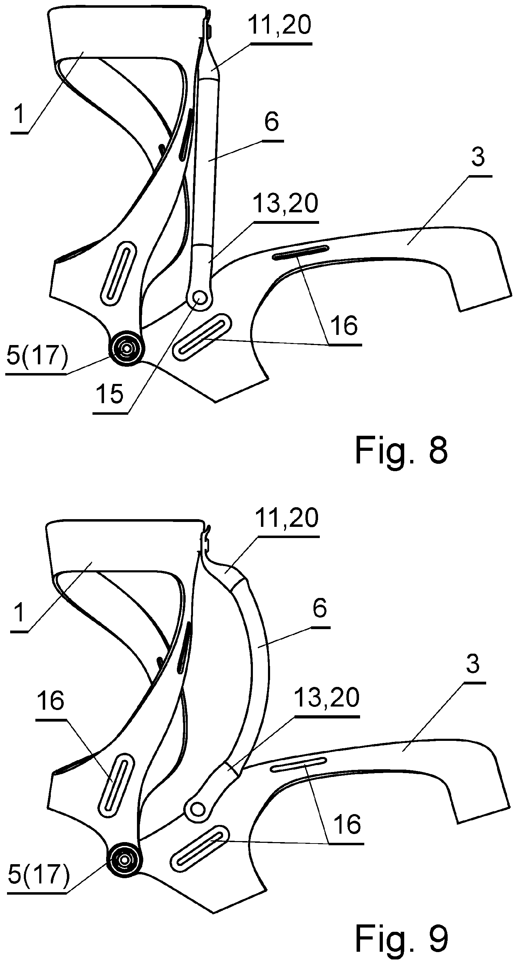

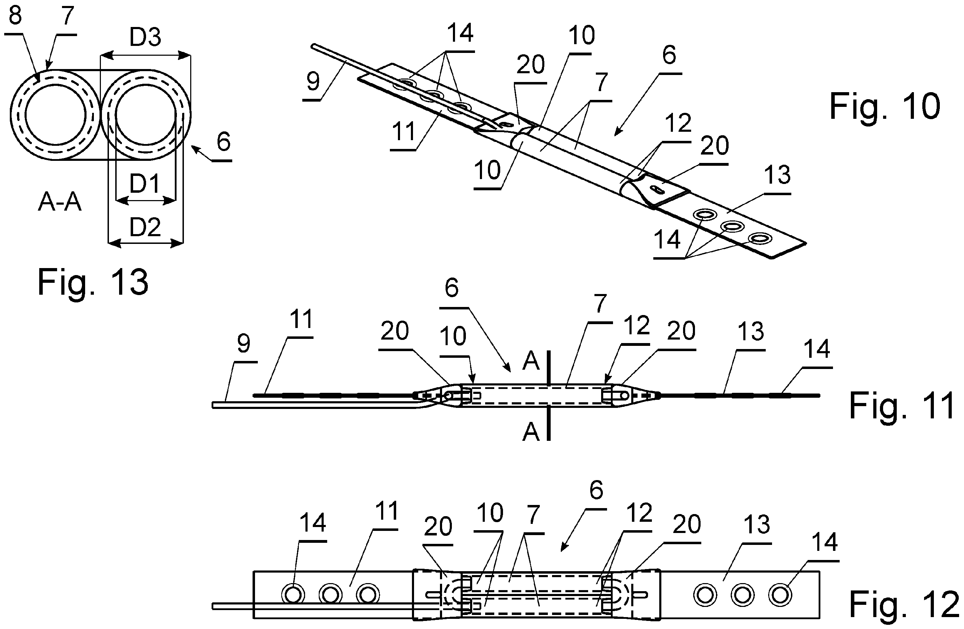

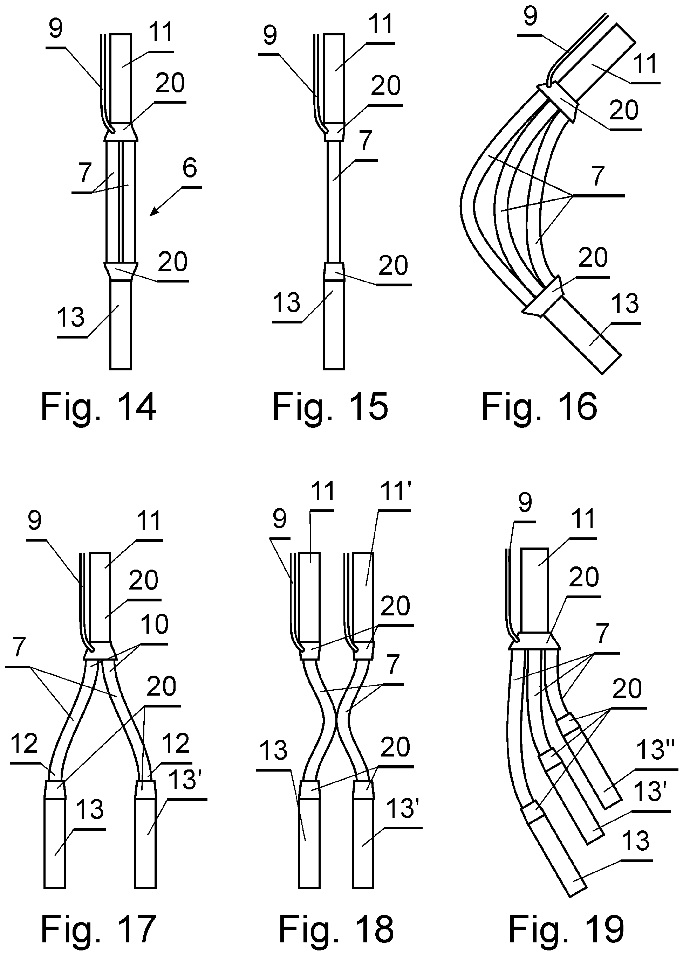

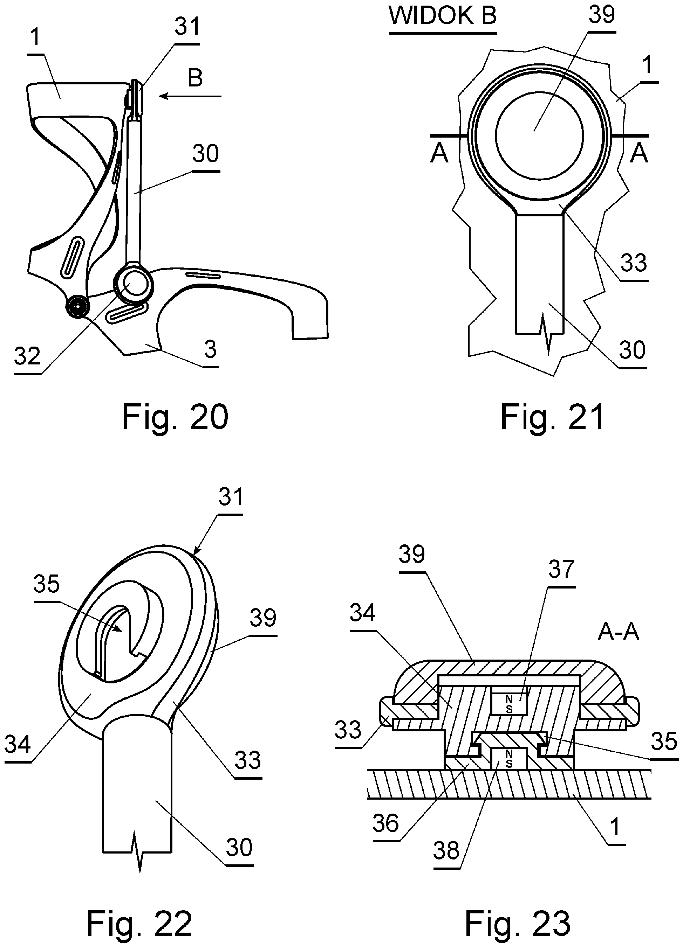

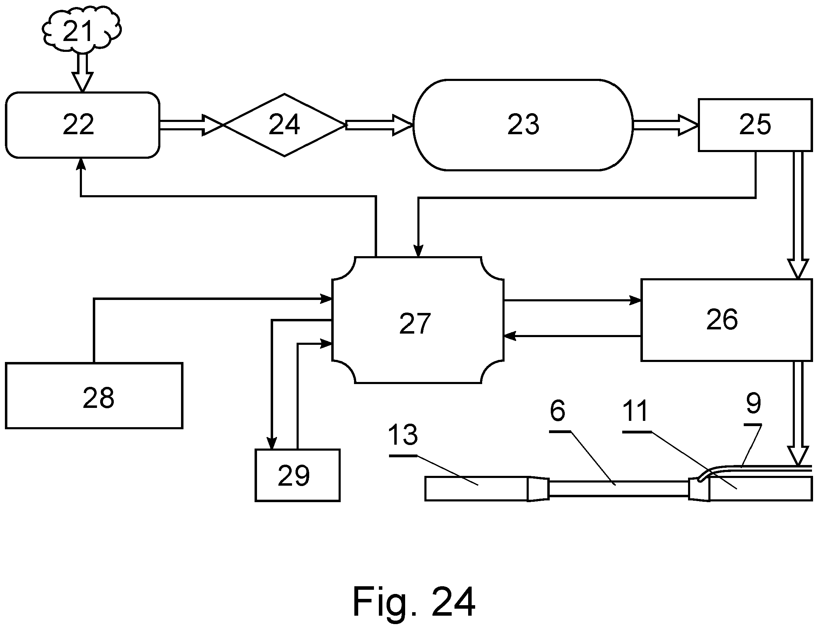

The invention from the exemplary embodiment was shown schematically on the drawing, where Fig.1 shows an axonometric view of the exoskeleton for an arm and forearm in a free state, while Fig.2 shows the exoskeleton of Fig.1 positioned on a limb in a side view. Fig.3 shows the exoskeleton of Fig.1 in a front view, while Fig.4 shows the exoskeleton of Fig.3 with both holders in a flared state, i.e. allowing it to be placed on a limb. Fig.5 shows the exoskeleton of Fig.1 in extension of the limb and Fig.6 shows the exoskeleton of Fig.5 in a side view. Fig.7 shows an enlarged view a portion of a view of Fig.5. Fig.8 shows the exoskeleton in a side view with maximum limb flexion, and Fig.9 shows the same exoskeleton with an unintentional pressure increase in the artificial pneumatic muscle. Fig.10, Fig.11, Fig.12 and Fig.13 show the two-chamber artificial pneumatic muscle used in the exoskeleton of Fig.1 in axonometric, side, top and cross-sectional views, respectively. Figures from Fig.14 to Fig.19 show schematically six different configurations of the pneumatic artificial muscle. Figures from Fig.20 to Fig.23 show without a scale details of the structure of an alternative pneumatic muscle attachment means, wherein Fig.20 shows the empty exoskeleton of Fig.2 to which the pneumatic muscle is attached by alternative means, Fig.21 shows a fragmentary view of a pneumatic muscle with such an alternative attachment means detached from the exoskeleton, Fig.22 shows an axonometric view of the T-slot of the pneumatic muscle of Fig.21 while Fig.23 is a cross-sectional view through this alternative attachment of the pneumatic muscle to the exoskeleton. Fig.24 shows an exemplary schematic diagram of an exoskeleton control system.

The exemplary exoskeleton according to the invention is adapted to support the movement of the forearm of one human hand. The exoskeleton has the first holder 1, put on the arm 2, the second holder 3 put on the forearm 4 and two joints 5 pivoting together the holders 1 and 3. Two pneumatic artificial muscles 6 are stretched between the holders 1 and 3. Each artificial muscle 6 includes two parallel flexible tubular working chambers 7 with an inner diameter D1 of 8 mm and a wall thickness of 2 mm, which gives an outer diameter D3 of chamber 7 of 12 mm. In practice, the inner diameter D1 of the working chamber 7 may range from 2 to 20 mm. The working chambers 7 are made in a known manner, for example according to the method disclosed in publication PL229119B1. The ch ambers 7 described here are molded from HCR silicone with a Shore A hardness of 10, but the chambers 7 molded from silicone with a hardness of 20 according to the same scale can also find practical application. The chambers 7 length in a free state is 80 mm. During moulding of the chamber 7, a helical braid 8 made of a non-stretchable polyester thread with a thickness of 0,25 mm was embedded in its wall. The axis of the braid 8 coincides with the axis of the chamber 7, its diameter D2 is 10 mm and the pitch of the braid 8 is from 0.3 mm to 1 mm. Each chamber 7 is blinded at both its ends, wherein the interiors of the two chambers 7 forming one muscle 6 are connected to each other at both ends, and a polyurethane pressure supply tube 9 with an outer diameter 3 mm is inserted into one of the plugs. The first ends 10 of the two chambers 7 forming one muscle 6 are connected to the first attachment means 11 and the second ends 12 of said chambers 7 are connected to the second attachment means 13. The attachment means 11 and 13 are in the form of a slender and unstretchable tape sections, with at least one opening 14 for a catch 15 located on the exoskeleton holder. The first 1 and second 3 holders are made of PA12 polyamide by 3D printing. This technology allows to easily make holders 1 and 3 closely matched to the user's anatomy. The material constituting these holders is the so-called semi-elastic material, characterized by a Young's modulus ranging from 1500 to 1900 MPa, whereby the exoskeleton according to the invention behaves in a more anatomically natural way than known exoskeletons during limb movement. Both holders 1 and 3 are in the form of an openwork, side-open sleeve with an average thickness of about 2.5 mm, surrounding the upper arm 2 and forearm 4, respectively. The material described above allows for an elastic lateral extension of both holders 1 and 3 (Fig.4) and insertion of the arm 2 and the forearm 4 through the resulting gaps. When the load spreading the holders 1 and 3 is released, the latter return to their original position, surrounding the assisted limb part. In order to avoid unintentional opening of any of the holders 1 and 3 during the operation of the exoskeleton, each of them is secured against it by means of Velcro straps, not shown in the drawing, threaded through the longitudinal holes 16 in the holders 1 and 3. The holders 1 and 3 are articulated with each other in the elbow area, by means of two ball joints 17, made of plastic under the trade name Iglidur J. This connection allows the natural movement of the hand in the elbow as it has one full degree of freedom (arm flexion) and two degrees of freedom limited by the range of mobility of the joints 17 and flexibility of the material when rotating the forearm 4. To increase the safety of use of the exoskeleton, at the stage of designing it for an individual user, end stops 18 and 19 are inserted into the joints to prevent excessive bending and extension of the arm.

The length of the pneumatic muscles 6 and the elasticity of the material of the working chamber 7 cause that in the resting state, i.e. in the absence of pressure, the arm supported by the exoskeleton is bent at the elbow at an angle of approximately 90 degrees (Fig.2). As the pressure in the chambers 7 increases, the hand in the exoskeleton falls under its own weight until it is fully extended or the stop 18 is achieved. This is because the braid 8 prevents the radial distending of the chamber 7 and the action of the pressure against the elastic force of the material of the chamber 7 only results in its elongation and, consequently, the elongation of the entire muscle 6. In other words, the artificial muscles 6 act in the opposite way to natural muscles or typical pneumatic actuators because in the absence of stimulation they are in the state of the greatest contraction and get flabby with increasing stimulation (pressure increase). In the described embodiment, the ends of the parallel chambers 7 of one muscle 6, are embedded in silicone plugs 20, in which also embedded are polyester bands 11 and 13 with three fixing holes 14 secured with metal eyelet rivets. The openings 14 make it very easy to quickly adjust the characteristics of the exoskeleton to the patient's needs, especially in the initial period of use. Both plugs 20 have a pneumatic channel connecting the interiors of both chambers 7 to each other, and one of them additionally has a compressed air inlet channel with a pipe 9 plugged into it, supplying compressed air from the control system described below.

A schematic diagram of the exoskeleton control system according to the invention is shown in Fig.24. Pneumatic muscles 6 are supplied with compressed air taken from the atmosphere 21 by the compressor 22 and fed by it to a pressure tank 23 through a check valve 24. At the outlet of the tank 23 there is a pressure sensor 25 and a regulating valve 26 responsible for the pressure in the artificial muscle 6. This system is managed by a controller 27, which monitors the pressure in the tank 23 by means of the pressure sensor 25 and activates the compressor 22 as necessary. The pressure in the described control system does not exceed the value of 3 bar, the operating pressure for the artificial muscles 6 being in the range from 0 to 2.5 bar. The controller 27 also receives a control signal from the manipulator 28 and the regulating valve 26 cooperates with the controller 27 in a feedback circuit. Thanks to this, it is possible to precisely adjust the air pressure in the muscle 6 to the signal from the manipulator 28. The manipulator 28 communicates to the controller 27 information about the force exerted by the patient on the manipulator and its return. Movement of the manipulator 28 in one direction causes a pressure drop in the artificial muscle 6 and, consequently, raising the forearm 4 under the influence of the elastic forces of the working chambers 7 material. Movement of the manipulator 28 in the opposite direction causes the pressure in the working chamber 7 to increase, as a result of which they extend and the forearm 4 falls under its own weight, even to full extension, wherein in this embodiment the speed of the exoskeleton movement, i.e. the speed of raising or lowering the forearm 4 depends on the value of the force applied by the patient to the manipulator 28. By ceasing the action on the manipulator 28, the pressure applied to it is maintained, thereby maintaining the desired position of the forearm 4. As shown in Fig. 9, the invention protects the patient from the unexpected pressure increase in the chambers 7 of the artificial muscles 6, since the high pressure only loosens the muscles 6. It is also possible to use two manipulators 28 activated by different parts of the body, where excitation of one manipulator causes the arm to bend and the other to extend. More complex sequences of movement along several axes, possible to be done with a shoulder, for example, can be realized using more than two manipulators, the effect of which on the individual muscles 6 is weighted. The design of the manipula- or 28 enables the control of that part of the patient's body in which the natural motor functions are at least partially preserved, e.g. the shoulder, finger, jaw or leg.

The function of the manipulator 27 can also be performed by a device with a touch screen, for example a typical smartphone connected wirelessly via a Bluetooth link to the controller 27, after installing a dedicated application on this device. In such an example, the differentiation of the pressure force can be replaced by moving the virtual slider on the smartphone screen.

In the example described, the controller 27 can handle up to five manipulators 28 and up to five regulating valves 26, i.e. a single control system can control up to five exoskeletons supporting a single patient. The control system has its own autonomous power supply and the communication interface 29 of the controller 27. The interface 29 has a display and buttons as well as a USB connector for connecting the controller 27 to a computer. The plugs 20 may be adapted to be connected to more than two working chambers 7 and the chambers 7 themselves may be of different lengths. This allows to build pneumatic artificial muscles similar to natural muscles, including those with more than two attachments, i.e. the equivalents of natural quadriceps muscles, and to adjust the total thickness of the artificial muscle to individual needs by deciding on the course of its individual working chambers 7. The drawing shows schematically various examples of artificial muscle configurations that can be used in the exoskeleton according to the invention. Fig.14 shows a two-chamber biceps muscle being an equivalent to the artificial muscle 6 described above, Fig.15 shows a one-chamber biceps muscle, and Fig.16 shows a biceps muscle with three non-parallel working chambers. Fig.17 shows an example of a two-chamber triceps muscle having the first attachment means 11 and two second attachment means 13 and 13'. Fig.18 shows an example of artificial quadriceps muscle with the two first attachment means 11 and 11' and the two second attachment means 13 and 13', and Fig.19 shows another variant of the artificial quadriceps muscle, with the three second attachment means 13, 13' and 13".

In another embodiment, the pneumatic muscle 30 is provided with alternative first 31 and second 32 attachment means. The pneumatic muscle 30 has at both its ends silicone plugs 33, functionally analogous to the plugs 20 of the pneumatic muscle 6. A blind seat 34 with a T-slot 35 is mounted in the plugs 33. The axis of the channel 35 coincides with the pneumatic muscle 30 axis of action. When mounting the muscle 30, a round mushroom-shaped catch 36 engages in the T-slot 35, functionally equivalent to the catch 15. The circular contour of the catch 36 allows for a free rotation of the seat 34 and the closure on one side of the T-slot 35 allows the muscle force to be transferred to the holders 1 and 3 of the exoskeleton. Free rotation of the seat 34 around the catch 36 improves the smooth operation of the pneumatic muscles and allows them to be attached and disassembled multiple times without noticeable wear of the attachment means. Both the seat 34 and the catch 36 have small permanent magnets 37 and 38 that automatically position the seat 34 on the catch 36 and prevent the pneumatic muscle 30 from self-detaching from the exoskeleton holders 1 and 3, for example during storage. The attraction force of the magnets 37 and 38 is, however, so low that the seat 34 can be manually slipped off the catch 36, if necessary, even by a disabled person. In the described example, the plug 33 is made of silicone with a hardness of 55 Shore A and has a hole for the seat 34. The part of the seat 34 protruding from this opening has a thread on which the pressure cover 39 is screwed. The threaded connection between the seat 34 and the cover 39 ensures a strong connection of the seat 34 with the muscle 30 and the easy replacement of the seat 34. The small-size catch 36 can be easily attached to the holders 1 and 3 of the exoskeleton and, if necessary, transferred to a more appropriate one from the point of view of specific limb motor skills.

## Claims

1. An exoskeleton comprising the first handle for the first body part, the second handle for the second body part, an articulated connection of both holders, at least one pneumatic artificial muscle, including at least one flexible working chamber with a non-stretchable braid and connecting both holders at its ends, and adjustable overpressure source supplying the interior of the working chambers of the artificial muscle, **characterized in that** the working chamber (7) of the artificial muscle (6) is in the form of a round tube section made of a flexible material, blinded at both ends, the braid (8) is embedded in the side wall of the working chamber (7) and is made of a non-stretchable thread running along a helical line with an axis coinciding with the axis of the working chamber (7), the diameter (D2) of the braid (8) is from 100% to 200% of the inner diameter (D1) of the working chamber (7), the pitch of the braid (8) is from 3% to 30% of its diameter (D2) and the thickness of the braid (8) thread is from 1% to 5% of its diameter (D2), the outer diameter (D3) of the working chamber (7) is from 100% to 200% of the diameter (D2) of the braid (8), and the length of each working chamber (7) in a free state is from 100 to 6000% of the diameter (D2) of the braid (8), while the hardness of the plastic forming the working chamber (7) is from 10 to 30 according to the Shore A scale.

2. The exoskeleton according to claim 1, **characterized in that** the first holder (1) is in the form of an openwork, side-open sleeve surrounding the arm (2), the second holder (3) is in the form of an openwork, side-open sleeve surrounding the forearm (4), both holders (1, 3) are made of elastic plastic and connected to each other by two ball joints (17), located in the elbow area.

3. The exoskeleton according to claim 2, **characterized in that** the plastic material of its holders (1, 3) has a Young's modulus ranging from 1500 to 1900 MPa.

4. The exoskeleton according to claim 3, **characterized in that** the plastic material from which the holders (1, 3) are made is polyamide.

5. The exoskeleton according to claim 2 or 3 or 4, **characterized in that** it comprises two artificial muscles (6), each artificial muscle (6) has at least two parallel, substantially identical, working chambers (7), the interiors of which are connected to each other, the first ends (10) of the working chambers (7) of one muscle (6) are connected to the first attachment means (11), securing the muscle (6) to the first holder (1), and the second ends (12) of the working chambers (7) of one muscle (6) are connected to the second attachment means (13), securing the muscle (6) to the second holder (3).

6. The exoskeleton according to claim 2 or 3 or 4, **characterized in that** it has at least two artificial muscles, each of the artificial muscles has at least two working chambers (7), the interiors of which are connected to each other, the first ends (10) of the working chambers (7) of one muscle (6) are connected to the first attachment means (11), securing the muscle (6) to the first holder (1), and the other ends (12) of the working chambers (7) of one muscle have its own separate second attachment means (13, 13') securing the muscle to the other holder (3).

7. The exoskeleton according to claim 5, **characterized in that** each artificial muscle (6) has two working chambers (7) made of silicone with hardness 10 Shore A, the inner diameter (D1) of each working chamber (7) is 8 mm, its outer diameter (D3) is 12 mm, and its length in a free state is from 40 to 120 mm, whereas the diameter (D2) of the braid (8) of each chamber (7) is 10 mm.

8. The exoskeleton according to claim 5 or 6 or 7, **characterized in that** the first (31) and the second (32) attachment means are in the form of a seat (34) with a blind T-slot (35), into which slot (35) a circular mushroom-shaped catch (36) is received, located on the holder (1,3), wherein the T-slot (35) is closed on the side of the action of this muscle (30), and the axis of this slot (35) coincides with the artificial muscle (30) axis of action.

9. The exoskeleton according to any of claims from 2 to 8, **characterized in that** the articulated connection (17) of the first (1) and the second (3) holder has a movement stop (18, 19), and each holder (1, 3) has strips preventing it from self-detaching from the limb (2, 4) it surrounds.
